# EUROPEAN PATENT APPLICATION

(11) **EP 3 272 218 A1**
(43) Date of publication of application: **24.01.2018**
(21) Application number: 16186648.8
(22) Date of filing: 31.08.2016
(51) Int. Cl.: A01P 7/04, A01N 37/18, A01N 43/40, A01P 17/00

(54) **CHEMICAL COMPOSITIONS THAT PRESENT SYNERGIC EFFECT FOR REPELLENCY OF INSECTS IN PERSONAL HYGIENE PRODUCTS, COSMETICS FOR HUMAN USE, IN ANIMALS AND ON INANIMATE SURFACES IN GENERAL**

(30) Priority: 19.07.2016 BR 102016016720
(71) Applicant: Universal Chemical Ltda, 18225-000 Sarapul, SP (BR)
(72) Inventor: Barros, Cassio Tadeu de Souza, 18225-000 Sarapui, SP (BR)
(74) Representative: Kador & Partner

(57) **Abstract**

Chemical compositions or mixture of components are described herein which, in combination and in diverse proportions with each other, present synergic effect, increasing the potential of insect repellent power, time of protective action and bestowing new insecticidal action, in personal hygiene products among others, as insect repellent among others. The synergic mixture is composed of 1-piperidinecarboxylic acid, 2(2-hydroxyethyl)-, 1-methylpropyl ester (Icaridin - CAS 119.515-38-7), N,N-dimethyl-3-methylbenzamide (DEET - Diethyltoluamide - CAS 134-62-3) combined with 3-Phenoxybenzyl 3-(2,2-dichlorovinyl)-2,2-dimethylcyclopropanecarboxylate (Permethrin - CAS 52.645-53-1) and/or 5-[[2(2-butoxyetoxy)etoxy]methyl]-6-propyl-1,3-benzodioxole (Piperonyl Butoxide - CAS 51-03-6). These mixtures of chemical compounds in diverse proportions, considering two or more of these cited components, present capacity of repelling insects on skin human, in animals or any other inanimate surface, superior to the already known capacity of repellancy of Icaridin and DEET individually and, attributes new insecticidal characteristics to the insect repellent products.

## Description

This patent process complies with requirements in the field of inventive activity, presenting technological advancements on insect repellent products in general, currently distributed in the world market, which can be applied to large-scale industrialization.

Chemical compositions or mixture of components are described herein which, in combination and in diverse proportions with each other, present synergic effect, increasing the potential of insect repellent power, time of protective action and bestowing new insecticidal action, in personal hygiene products among others, as insect repellent among others.

The synergic mixture is composed of 1-piperidinecarboxylic acid, 2(2-hydroxyethyl)-, 1-methylpropyl ester (Icaridin - CAS 119.515-38-7), N,N-dimethyl-3-methylbenzamide (DEET - Diethyltoluamide - CAS 134-62-3) combined with 3-Phenoxybenzyl 3-(2,2-dichlorovinyl)-2,2-dimethylcyclopropanecarboxylate (Permethrin - CAS 52.645-53-1) and/or 5-[[2(2-butoxyetoxy)etoxy]methyl]-6-propyl-1,3-benzodioxole (Piperonyl Butoxide - CAS 51-03-6)

These mixtures of chemical compounds in diverse proportions, considering two or more of these cited components, present capacity of repelling insects on skin human, in animals or any other inanimate surface, superior to the already known capacity of repellancy of Icaridin and DEET individually and, attributes new insecticidal characteristics to the insect repellent products.

### HISTORIES AND BENEFITS OF THE INVENTION

In many regions of the world, hazardous diseases, such as malaria, yellow fever, microencephalia among others, are transmitted by mosquitoes, crane flies and other insects. Statistics show that a person dies every day in the world every 30 seconds due to complications generated by insect bites. Malaria alone causes about 3 million deaths per year in the entire world.

In the United States and Europe, disease-transmitting insects are more rare but, the movement of tourists in these countries, transports the virus and raises the risk index of acquiring a disease typical of more tropical countries.

Diverse attempts of controlling or eliminating the proliferation of insects have not been successful. Other methods such as vaccination have been the most commonly used tool, however, the prevention through the use of repellents has grown rapidly throughout the world.

Male insects survive only by consuming plant nectar. Only female insects bite humans and animals due to their need of obtaining protein to generate an egg cell and afterwards, the embryo.

With the intention of locating their potential sources of blood or protein, female insects have a complex sensory system for identification of their potential donors. These sensors detect the presence of moisture, heat, carbonic gas and chemical components such as amino acids, lactic acid, ammonium, among other substances excreted by the human skin.

Insects are attracted to their potential protein donors through the smell of these components which are detected by their sensors. Upon locating the donor, the insect settles on the skin and introduces its proboscis (needle) to inject a secretion that dilates the blood vessels, while at the same time, sucks the blood through a second channel.

Insect repellent products, once deposited on the skin, form a layer with odors that act directly on the sensory system of the female insect, confounding the signals that identify a protein source such as the blood.

Chemically, repellent compounds are derived from amides, alcohols, esters and ethers which present a low evaporation rate at ambient temperature, remaining for a longer time on the skin, providing greater protection to the repellent product user.

The most renowned active ingredients with capacity of repelling insects are:
A) 1-piperidinecarboxylic acid, 2-(2-hydroxyethyl)-, 1-methylpropyl ester (Icaridin - CAS 119.515-38-7)
B) N,N-dimethyl-3-methylbenzamide (DEET - Diethyltoluamide - CAS 134-62-3)

The compound Icaridin has advantages over DEET. The former presents more moderate allergic reactions when in contact with the skin and less toxicity to human metabolism than DEET. Icaridin also showed to be more biodegradable than DEET, presenting less environmental impact, recognizably in the marine environment.

By analyzing the capacity of repelling insects in the course of time, the compound Icaridin also presents better performance than DEET.

| **Concentration of the Active ingredient in the repellent** | **Repellent Capacity Length of Protection (hours)** | |
|---|---|---|
| | **Icaridin** | **DEET** |
| 5% | 1.5 | 1 |
| 10% | 4 | 2 |
| 15% | 7 | 4 |
| 20% | 10 | 6 |
| 50% | - | 10 |

In spite of the clear advantages of Icaridin over DEET, the worldwide availability for supply of Icaridin, does not permit yet the complete replacement of DEET in insect repellent products. For this reason, the two active ingredients still live together in the world market.

This invention, through the use of a synergic mixture of these two active ingredients with Permethrin and Piperonyl Butoxide, permits the employment of lower concentrations of Icaridin and DEET in repellent products, maintaining the same or longer time of protection, by adding the insecticidal capacity of Permethrin to the final product.

The employment of lower concentration of the active ingredients will contribute to a more rationalized consumption of Icaridin and DEET, as well as it will lead to lesser long-term environmental impact.

### DETAILED DESCRIPTION OF THE PREFERRED MODES OF THE INVENTION

For the purposes of this invention, "insects" mean all flying or creeping insects that can bite the human or animal skin in order to reach the subepithelial tissue or bloodstream, aiming to obtain nutrients for its subsistences.

For the purposes of this invention, "insect repellent" means any product in liquid, spray, lotion, cream, aerosol, powder, gel or solid form that has the capacity of driving away insects from contact with the human or animal skin.

For the purposes of this invention, "length of protection against insect bites" means the length of time that the insect repellent, after it has been applied on the human or animal skin, is able to maintain its capacity of repelling insects, being effective in preventing their bites.

For the purposes of this invention, "active ingredient" means the chemical substance that constitutes the insect repellent and provides this, the best or 5/7 lesser capacity of repelling insects for the pre-established time of protection on the repellent product's label.

For the purposes of this invention, "potential-intensifying agents" mean chemical substances that increase the capacity of the active ingredient to act on the insects.

For the purposes of this invention, "synergic mixture" means a composition of active ingredients and potential-intensifying agents that, when they act jointly, presents greater insect repellent capacity than the individual capacity of their components, which is determined by the % of active ingredient employed in the insect repellent formulation when compared with the obtained length of protection against bites.

The chemical compounds and their proportions that characterize this invention, use diverse active ingredients and potential-intensifying agents existing in the market, however it is focused on the synergic mixture between them in quantitative and qualitative proportions.

The chemical compounds included in this synergic mixture for repelling insects, are:
**A)** 1-piperidinecarboxylic acid, 2-(2-hydroxyethyl)-, 1-methylpropyl ester (Icaridin - CAS 119.515-38-7)
**B)** N,N-dimethyl-3-methylbenzamide (DEET - Diethyltoluamide - CAS 134-62-3)
**C)** 3-Phenoxybenzyl 3-(2,2-dichlorovinyl)-2,2-dimethylcyclopropanecarboxylate (Permethrin - CAS 52.645-53-1)
**D)** 5-[2-(2-butoxyetoxy)etoxy]methyl]-6-propyl-1,3-benzodioxole (Piperonyl Butoxide - CAS 51-03-6)

Icaridin and DEET are employed in this synergic mixture as active ingredient. Their capacities of repelling insects are potentially intensified by the agents Permethrin and Piperonyl Butoxide, conceding length of protection against insect bites equal to or greater than 10 hours after 6/7 application of the product on the skin, even when these active ingredients are used in quantities lower than those pre-established by their technical literatures. The table below, shows the data obtained in the conducted tests.

| **Active Ingredient** | **% concentration in the insect repellent** | **Length of protection against insect bites** |
|---|---|---|
| Icaridin | 10% | 4 hours |
| | 20% | 10 hours |
| DEET | 7% | 2 hours |
| | 50% | 10 hours |
| Icaridin + Permethrin + Piperonyl Butoxide | 1% to 10% | Greater than 10 hours |
| DEET + Permethrin + Piperonyl Butoxide | 1% to 20% | Greater than 10 hours |
| Icaridin + DEET + Permethrin + Piperonyl Butoxide | 1% to 20% | Greater than 10 hours |

### SUMMARY OF THE INVENTION

This invention provides chemical compositions for insect repellent products for human or animal use or use on inanimate surfaces which provide a capacity of repelling insects superior to typical market products, exclusively formulated with Icaridin or DEET.

The combination of Icaridin or DEET with Permethrin in the presence of Piperonyl Butoxide, causes the length of protection against insect bites granted by the capacity of repelling insects of these substances to be superior, even by considering % of participation of these components lower in the products' formulations.

DEET in concentrations of 10% to 20%, presents capacity of repelling insects for 1 to 2 hours. Now Icaridin in concentrations between 20 to 25% is able to protect the user against insect bites for up to 10 hours. For DEET to reach this same length of protection, it is necessary for it to be applied at a concentration of 50%.

When the insect repellent product composition presents these substances in the presence of 1% to 10% Permethrin and Piperonyl Butoxide, so as to reach the same length of time (10 hours) against insect bites, concentrations lower than 10% of Icaridin or DEET are necessary.

The chemical compositions now invented, can also receive addition of compounds that grant moisturizing capacity to dry skin, preventing the skin from drying due to contact with these components.

Alcohol compounds and isopropyl myristate can also be added to reduce allergic reactions at the site of the bite, minimizing itching.

These mixtures of insect repellent agents, can also absorb skin protecting components against UVA and UVB rays such as Diethylamino Hydroxybenzoyl Hexyl Benzoate, Octyl triazone and Titanium Dioxide, granting to the insect repellent product, additional characteristics, making the final product 2 in 1, being an insect repellent and a sunscreen at the same time.

The presence of Permethrin in the synergic mixture, grants the insect repellent, an additional insecticidal capacity to typical products of this market segment.

## Claims

1. "Chemical composition for insect repellents," for all purposes in human or animal use or use on inanimate surfaces, characterized for containing the mixture of the 4 components listed below, in any proportion:
**A)** 1-piperidinecarboxylic acid, 2-(2-hydroxyethyl)-, 1-methylpropyl ester (Icaridin - CAS 119.515-38-7);
**B)** N,N-dimethyl-3-methylbenzamide (DEET - Diethyltoluamide - CAS 134-62-3);
**C)** 3-Phenoxybenzyl 3-(2,2-dichlorovinyl)-2,2-dimethylcyclopropanecarboxylate (Permethrin - CAS 52.645-53-1); and
**D)** 5-[2-(2-butoxyetoxy)etoxy]methyl]-6-propyl-1,3-benzodioxole (Piperonyl Butoxide - CAS 51-03-6);
which are solubilized in vehicles such as water, alcohols and glycols, which can contain thickeners, serous materials or resins, which can contain compounds for skin protection against UVA and UVB sun rays, which can contain anti-allergy agents, such that the final presentation of this mixture can be in liquid, paste, powder or solid forms.

2. "Composition", in accordance with claim 1, characterized for containing components:
**A)** 0% to 35% by weight of Icaridin (CAS 119.515-38-7);
**B)** 0% to 60% by weight of DEET (CAS 134-62-3);
**C)** 0% to 10% by weight of Permethrin (CAS 52645-53-1); and
**D)** 0% to 10% by weight of Piperonyl Butoxide (CAS 51-03-6).

3. "Composition", in accordance with claims 1 and 2, characterized for presenting synergy between their components in relation to the capacity of repelling insects; this mixture of components presents capacity superior to the individual capacities of each of its components, permitting it to use less quantity of each component in order to reach the same length of protection against insect bites on the part of the user of this composition or product.

4. "Composition", in accordance with claims 1 and 2, characterized for having insecticidal action together with its insect repellent power, granted by the synergic composition of Permethrin and Piperonly Butoxide.

5. "Composition", in accordance with claims 1 and 2, characterized for being used in insect repellents for skin and clothes, in personal hygiene products, cosmetics, for human use or in animals or on inanimate surfaces.

6. "Composition", in accordance with claims 1 and 2, characterized for containing addition of Aliphatic Alcohols and Isopropyl Myristate (CAS 110-27-0), leading to reduction of itching generated at the site of the skin where it was bitten.

7. "Composition", in accordance with claim 1, characterized for containing addition of skin protecting compounds against UVA and UVB sun rays based on Diethylamino Hydroxybenzoyl Hexyl Benzoate (CAS 302776-68-7), Octyl Triazone (CAS 88122-99-0) and Titanium Dioxide (CAS 13463-67-7).

8. "Composition", in accordance with claim 1, characterized for containing carbomer-based thickeners and polyglycols.

9. "Composition", in accordance with claim 1, characterized for containing fatty acid based serous materials and their derivatives, mineral oils, vegetable oils, paraffins and olefins.

10. "Composition", in accordance with claim 1, characterized for containing acrylic resins or extracted from any plant.

11. "Composition", in accordance with claim 1, characterized for containing moisturizing compounds for human or animal skin, based on vegetable or mineral oils, non-ionic surfactants, derived from stearic acid, glycols and other similar alcohols that present this capacity of moisturizing the skin.
